# EUROPÄISCHE PATENTSCHRIFT

(11) **EP 0 282 018 B1**
(45) Veröffentlichungstag und Bekanntmachung des Hinweises auf die Patenterteilung: **05.07.1995**
(21) Anmeldenummer: 88103668.5
(22) Anmeldetag: 09.03.1988
(51) Int. Cl.: C07K 14/78, C07K 1/113

(54) **Verfahren zur Isolierung von Basalmembranproteinen aus menschlichen und tierischen Geweben**
Process for isolating basal membrane proteins from human or animal tissues
Procédé pour isoler des protéines de membrane de base de tissus humains et animaux

(30) Priorität: 13.03.1987 DE 3708198
(43) Veröffentlichungstag der Anmeldung: 14.09.1988
(73) Patentinhaber: HOECHST AKTIENGESELLSCHAFT, 65926 Frankfurt am Main (DE); Max-Planck-Gesellschaft zur Förderung der Wissenschaften e.V., D-37073 Göttingen (DE)
(72) Erfinder: Brocks, Dietrich, Dr., D-6200 Wiesbaden (DE); Timpl, Rupert, Dr., D-8035 Gauting (DE); Paulsson, Mats, Dr., D-8033 Martinsried (DE)

(56) Entgegenhaltungen:
- EP-A- 0 198 259
- THE JOURNAL OF BIOLOGICAL CHEMISTRY, Band 254, Nr. 19, 10. Oktober 1979, Seiten9933-9937, US; R. TIMPL et al.: "Laminin - A glycoprotein from basementmembranes"
- R.K. SCOPES: "Protein purification, Springer Advanced texts in chemistry",
- 1987, Seiten 33-38,250-251, Springer Verlag, New York, US
- P.M.B. WALKER: "Chambers science and technology dictionary", 1988, Seite 286,Chambers, Cambridge, GB
- Eur.J.Biochem. 161 (1986) 455-464
- Techniques in Lipid Membr. Biochem. B408 (1982) 1-22
- Connective Tissue Research 14 (1985) 31-40

## Beschreibung

Laminin, Nidogen und BM40 sind Glykoproteine, denen eine Schlüsselrolle in der Organisation von Basalmembranen und bei der Wechselwirkung von Zellen mit Basalmembranen zugeschrieben wird [Paulsson, M. et al. Eur. J. Biochem. 161, 455-464 (1986), R. Timpl, M. Dziadek, Int. Rev. Exp. Pathol. 29, 1-112 (1986)]. So unterstützt Laminin das Auswachsen von Neuriten aus den Neuronen peripherer Nerven [Edgar, D. et al., EMBO J. 3, 1463-1467 (1984)] und deren Regeneration in situ [Madison et al. Exp. Neurol. 88, 767-772 (1985)].

Eine breitere Anwendung dieser Proteine wird bislang vor allem dadurch verhindert, daß keine geeigneten Verfahren zur Verfügung stehen, mit denen diese Proteine auf schonende Weise aus humanem Gewebe isoliert werden können. Das von Risteli und Timpl [Biochem. J. 193, 749-755 (1981)] beschriebene Verfahren behandelt die Isolierung des proteaseresistenten Laminin-Fragmentes P1 aus Humanplazenta. Dieses Fragment enthält nicht mehr die für die Wechselwirkung mit Nerven und Tumorzellen wichtige Domäne 8. Mit Hilfe des von Timpl et al. [J. Biol. Chem. 254, 9933-9937 (1979)] beschriebenen Verfahrens zur Isolierung von Laminin aus Tumorgewebe kann aus Humanplazenta kein Laminin solubilisiert werden und aus Tumorgewebe erhält man nach diesem Verfahren nur niedrige Ausbeuten.

Nach dem von Ohno et al. [Biochem. Biophys. Res. Com. 112, 1091-1098 (1983)] und Wewer et al. [J. Biol. Chem. 258, 12654-12660 (1983)] beschriebenen Verfahren benötigt man entweder denaturierende Bedingungen oder Proteolyse, um signifikante Mengen von Laminin oder Laminin Bruchstücke aus dem Gewebe zu extrahieren. Auch Nidogen oder BM40 können bisher nur unter denaturierenden Bedingungen isoliert werden [Dziadek et al., Eur. J. Biochem. 161, 455-464 (1985), Paulsson et al., Eur. J. Biochem. 156, 467-478, (1986)].

Demgegenüber ist es Aufgabe der vorliegenden Erfindung, auf schonende Weise Basalmembranproteine zu isolieren, die als Antigen zur Herstellung hochspezifischer Antiseren bzw. Antikörper eingesetzt werden.

Überraschenderweise kann man aus waßriger Lösung in Gegenwart eines Chelatbildners intakte Basalmembranproteine extrahieren und zwar in größeren Mengen als bisher möglich.

Die Erfindung betrifft somit die Verwendung von Chelatbildnern zur Isolierung von Basalmembranproteinen aus menschlichen oder tierischen Geweben, enthaltend die folgenden Schritte:
a) eine Vorextraktion des basalmembranhaltigen Gewebes mit einem Puffer, der die biologische Aktivität der Basalmembranproteine erhält sowie Proteaseinhibitoren enthält;
b) eine Extraktion der Basalmembranproteine aus menschlichen oder tierischen Geweben mittels eines Puffers, der die biologische Aktivität der Basalmembranproteine erhält sowie Proteaseinhibitoren und Chelatbildner enthält,
wobei die Vorextraktion oder die Extraktion nicht jedoch in Anwesenheit von Triton® X 100 erfolgt.

Die auf diese Weise erhältlichen Basalmembranproteine sind zur Herstellung hochspezifischer Antiseren oder Antikörper und damit zur Bestimmung der Basalmembranproteine in Körperflüssigkeiten und Gewebeextrakten geeignet.

Im folgenden wird die Erfindung detailliert, insbesondere in den bevorzugten Ausführungsformen, erläutert. Ferner wird die Erfindung in den Patentansprüchen definiert.

Mit Hilfe der vorliegenden Erfindung können alle menschlichen oder tierischen Gewebearten, in denen sich Basalmembranproteine befinden, wie z.B. Humanplazenta, menschliche oder tierische Tumorgewebe oder die Niere, extrahiert werden. Es kann zunächst eine Vorextraktion durchgeführt werden. Das Gewebe wird in einem für Basalmembranproteine physiologischen Puffer in Gegenwart von Proteaseinhibitoren homogenisiert und zentrifugiert. Bei dieser Behandlung werden bevorzugt leicht lösliche Proteine, wie Serumproteine und cytoplasmatische Proteine des Gewebes entfernt. Der Geweberückstand wird dann nochmals in einem physiologischen Puffer aufgenommen und in Gegenwart von Proteaseinhibitoren und chelatbildenden Agenzien homogenisiert und extrahiert. Geeignete Chelatbildner sind organische Polysäuren, wie z.B. EDTA, EGTA oder Citrat. Die Extraktion wird unter Zugabe von 1-500 mmol/l des Chelatbildners, bevorzugt 10-50 mmol/l, zweckmäßig unter Kühlung bei 0-15°C, bevorzugt 4-10°C, durchgeführt. Bei höheren Temperaturen ist eine Extraktion auch noch möglich, sie wird jedoch dann von störenden Abbaureaktionen begleitet. Die Extrakte enthalten die gewünschten Basalmembranproteine. Besonders bevorzugt werden erfindungsgemäß BM40, Nidogen und Laminin, zum Teil als nicht kovalenter Komplex, isoliert.

Die einzelnen Proteine werden aus dem Extrakt durch Molekularsiebchromatographie an geeigneten Säulen, wie z.B. Dextran, insbesondere Allyldextran vernetzt mit N,N'-Methylenbisacrylamid, oder Agarose, isoliert. Die Feinreinigung der Basalmembranproteine erfolgt je nach Protein an bevorzugt schwach basischen Ionenaustauschern mit anschließender Molekularsiebchromatographie bzw. weiterer Reinigung an Agarose oder Sepharose Säulen.

Auf diese Weise können Mengen von intakten Basalmembranproteinen isoliert werden, die mit herkömmlichen Isolierungsverfahren nicht erreicht werden.

Die aufgetrennten gereinigten Basalmembranproteine können dann jeweils als Antigen zur Herstellung hochspezifischer Antiseren bzw. Antikörper eingesetzt werden. Dies geschieht in üblicher Weise durch subcutane oder intramuskuläre Injektion an Versuchstieren, vorzugsweise an Kaninchen. Zweckmäßig wird dabei in Gegenwart von komplettem Freund'schen Adjuvans gearbeitet. Es können die in diesen Fällen üblichen Antigendosen angewendet werden. Die bevorzugte Dosis am Kaninchen ist 0,5 bis 1 mg pro Tier. Das gebildete Antiserum wird dann in der dem Fachmann bekannten Weise gewonnen und kann als solches verwendet werden. Es ist auch möglich, die im Serum vorhandenen Antikörper vorher noch zu reinigen. Eine bevorzugte Methode dabei ist die Affinitätschromatographie.

Das mit Hilfe der gereinigten Basalmembranproteine hergestellte hochspezifische Antiserum ermöglicht, die Bestimmung von Basalmembranproteinen in Körperflüssigkeiten, wie beispielsweise Serum, Urin etc. und Gewebeextrakten unter Anwendung der an sich bekannten immunologischen Nachweismethoden.

Es können bei diesen Bestimmungen sowohl die bekannten Radio-Immun-Assay-(RIA)-Varianten als auch die Enzym-Immun-Assay-Varianten und analoge Verfahren angewendet werden.

Die für die immunologische Bestimmung notwendige Markierung des Antigens kann in den für Proteinmarkierung bekannten Methoden erfolgen. Es wird bevorzugt radioaktiv-, enzym- oder fluoreszensmarkiert. Im Falle der radioaktiven Markierung wird als Radionuklid bevorzugt Jod 125 verwendet. Die Markierung kann dann nach der bekannten Chloramin T Methode (Int. Arch. Allergy 29, 185, 1966) erfolgen.

Bei diesen Nachweisreaktionen konkurriert das markierte Basalmembranprotein in bekannter Weise um den Antikörper, so daß im gebildeten Antigen-Antikörper-Komplex die Menge markierten Antigens umso geringer ist, je mehr nicht markierte Antigene in der zu bestimmenden Probe enthalten sind. Es kann entweder die Markierung des Komplexes, beispielsweise die Radioaktivität oder die Enzymaktivität, oder die Markierung des Überstandes nach Abtrennung des Antigen-Antikörper-Komplexes verwendet werden, um anhand einer Eichkurve, die mittels Proben bekannten Gehalts an Basalmembranprotein erstellt wurde, die in der zu untersuchenden Probe enthaltenen Mengen Antigen festzustellen. Es wird bevorzugt die Markierung im Komplex bestimmt.

Eine bevorzugte Ausführungsform der erfindungsgemäßen Methode besteht darin, die Abtrennung des mit dem spezifischen Antiserum gebildeten Antigen-Antikörper-Komplexes mit Hilfe eines zweiten Antikörpers durchzuführen. Bevorzugt wird dazu ein gegen Immunglobulin G der für die Gewinnung des spezifischen Antiserums verwendeten Tierart gerichteter Antikörper verwendet. Die Abtrennung des Antigen-Antikörper-Komplexes von der Lösung kann mit hierfür üblichen Methoden wie Fällung, Zentrifugation oder Abfiltrieren erfolgen. Alternativ hierzu wird das Antiserum bzw. der zweite Antikörper an einen festen Träger gebunden. Nach Abtrennung des Antigen-Antikörper-Komplexes wird dann die im Komplex enthaltene, bzw. die in der Lösung verbliebene Radioaktivität oder andere Markierung bestimmt.

Die Enzym-Immun-Assay-Variante zur Bestimmung von Basalmembranprotein kann auch dergestalt durchgeführt werden, daß der Antikörper gegen Immunglobulin G der betreffenden Tierart, z.B. vom Kaninchen, enzymmarkiert ist. Bei dieser Art des Enzym-Immun-Assay wird der nach Bildung des Antigen-Antikörper-Komplexes verbleibende Teil des Anti-Basalmembranprotein-Serum bestimmt, indem man diesen an trägergebundenes Basalmembranprotein bindet und anschließend mit enzymmarkierten Antikörpern gegen Immunglobulin G des Kaninchens zur Reaktion bringt. Die Menge gebundenen enzymmarkierten Antikörpers wird anschließend durch Messung der Enzymreaktion bestimmt und ist der unbekannten Menge des Basalmembranproteins in der Probe indirekt proportional.

In den folgenden Beispielen wird die Erfindung weitergehend erläutert.

### Beispiel 1

### Isolierung der Basalmembranproteine Laminin, Nidogen und BM40.

EHS-Tumorzellen wurden subcutan in C57Bl-Mäuse injiziert [Orkin et al. J. Exp. Med. 145, 204 (1977)].Nach 10-14 Tagen wird der Tumor geerntet und das anschließend gereinigte Gewebematerial bei -20°C gelagert. 75 g der gefrorenen Tumore wurden 1 min. in 1,5 l 0,15 M NaCl, 0,05 M Tris-HCl (pH 7,4) (TBS) mit 0,05 mM Phenylmethansulfonylfluorid (PMSF) und N-Ethylmaleimid (NEM) homogenisiert. Nach der Zentrifugation bei 8000 rpm über einen Zeitraum von 20 min. wurde der Überstand verworfen und der Rückstand nochmals in 375 ml des obengenannten Puffers unter Zugabe von 10 mM EDTA homogenisiert und im Kühlraum eine Stunde gerührt. Der Extrakt wurde nach oben angegebener Zentrifugation gewonnen und entweder sofort weiterverwendet oder bei -20°C gelagert. Die Ausbeute und Reinheit des gewünschten Produkts konnten weiter erhöht werden, wenn die Extraktionsschritte wiederholt wurden.

Eine aliquote Menge des TBS-EDTA Extrakts wurde über eine Agarose-(®Biogel A5m) oder eine Sepharose CL-6B-Säule (3 x 135 cm) gegeben, die in TBS mit 2 mM EDTA, 0,5 mM PMSF und 0,5 mM NEM äquilibriert worden war. Dabei kam es zur Aufspaltung in 3 bis 4 Fraktionierungsbereiche. Das zuerst mit dem Ausschlußvolumen eluierte Material enthält Laminin-Nidogen-Komplex und freies Laminin, die nachfolgenden Fraktionen enthalten freies Nidogen in intakter (MG 150 KD) und teilweise abgebauter Form (MG 130, 100 KD). Anschließend wird BM 40 eluiert.

Die Reinigung des Laminin erfolgt zunächst an einem schwach basischen Ionenaustauscher (DEAE Cellulose) mit 2 M Harnstoff in 0,05 M Tris/HCl (pH 8,6) unter Zugabe von Proteaseinhibitoren. Es wird mit Hilfe eines linearen NaCl-Gradienten (0, bis 0,4 M) eluiert. Bei diesem Schritt werden störende Verunreinigungen von Proteoglykan und Nukleinsäuren entfernt. Laminin wird schließlich durch Chromatographie an Sepharose CL-4B in 2 M Guanidinchlorid (pH 7,4) von restlichem Nidogen und kleineren Proteinen befreit.

Die Reinigung von Nidogen erfolgt zunächst an DEAE-Cellulose und Sepharose CL-4B wie bei Laminin beschrieben. Weitere Reinigungsschritte, wenn notwendig, beinhalten Chromatographie an CM-Cellulose mit 2 M Harnstoff, 0,02 M Na-Acetat (pH 4,8) und vernetztem Allyldextran (Sephacryl S200) mit 0.2 M NH₄HCO₃.

Die weitere Reinigung von BM40 erfolgt ebenfalls an DEAE-Cellulose mit 2 M Harnstoff, 0,05 M Tris (pH 8.6) durch Elution mit Hilfe eines linearen NaCl-Gradienten (0-0,4 M NaCl). BM40 eluiert bei 0,2-0,25 M NaCl und wird nach Konzentrierung über Sephacryl S200 mit 0,2 M NH₄HCO₃ endgültig gereinigt.

Die Tabelle zeigt beispielhaft, daß mit der schonenden Extraktion Mengen aus intakten Basalmembranproteinen, z. B. EHS-Tumor, extrahiert werden, die mit herkömmlichen Verfahren nicht erreicht werden können.

**Tabelle**

| Gefundenes Protein in mg pro g Feuchtgewicht | | | |
|---|---|---|---|
| | Laminin | Nidogen | BM40 |
| TBS-Extrakt | 0,98 | 0,05 | 0,07 |
| TBS/EDTA-Extrakt | 2,84 | 0,46 | 0,34 |
| 0,5 M NaCl | 0,39 | < 0,05 | nicht bestimmt |
| 6 M Guanidinchlorid | 0,83 | 0,30 | < 0,01 |

### Beispiel 2

### Herstellung des markierten Antigens

25 »g des nach Beispiel 1 isolierten Basalmembranproteins werden mit 0, 5 mCi Jod 125 nach der Chloramin T-Methode markiert und ungebundenes Jod durch Dialyse oder Gelfiltration an einem Polyacrylamid-Gel (z. B. ®Biogel P2, Pharmacia Fine Chemicals Inc.) entfernt.

### Beispiel 3

### Durchführung der immunologischen Bestimmung

Alle für die immunologische Bestimmung notwendigen Schritte werden in Gegenwart von 0,04% eines nichtionischen Detergens, wie z. B. Tween 20, ein polyethoxyliertes Sorbitan-Monolaurat, durchgeführt. Bindungskurven werden mit 1 ng markiertem, nach Beispiel 1 isolierten Basalmembranprotein bestimmt. Die Konzentration von Basalmembranprotein in einer unbekannten Probe von Serum oder anderen Körperflüssigkeiten wird in folgendem Inhibitionstest bestimmt:
Eine bestimmte Menge des spezifischen Antikörpers oder Antiserums wird mit der unbekannten Probe 16 h bei 4°C vorinkubiert und nach Zugabe von 1 ng markierten Antigens weitere 8 Stunden bei 4°C inkubiert. Danach wird ein Überschuß von Antikörpern gegen Kaninchen-Immunoglobulin G zugesetzt und nach weiteren 16 h bei 4°C das im Immunkomplex gebundene Antigen durch Zentrifugation abgetrennt. Die Inhibitionsaktivität der unbekannten Probe wird mit der Aktivität einer Standard-Konzentration nicht-markierten Antigens verglichen.

### Beispiel 4

### Herstellung des Antiserums

Kaninchen werden mit 0,5 bis 1 mg des nach Beispiel 1 isolierten Basalmembranproteins pro Tier immunisiert, indem die Antigenlösung, gemischt mit gleichen Volumen komplettem Freund'schen Adjuvans, subcutan oder intramuskulär injiziert wird. 4 Wochen nach der ersten Injektion wird eine gleiche Menge Antigenlösung, gemischt mit komplettem Freund'schem Adjuvans, subcutan oder intramuskulär injiziert. 4 bis 8 Wochen nach der 2. Injektion wird das Blut gewonnen und daraus nach Gerinnung das Antiserum erhalten.

## Patentansprüche

1. Verwendung von Chelatbildnern zur Isolierung von Basalmembranproteinen aus menschlichen oder tierischen Geweben, enthaltend die folgenden Schritte:
a) eine Vorextraktion des basalmembranhaltigen Gewebes mit einem Puffer, der die biologische Aktivität der Basalmembranproteine erhält sowie Proteaseinhibitoren enthält;
b) eine Extraktion der Basalmembranproteine aus menschlichen oder tierischen Geweben mittels eines Puffers, der die biologische Aktivität der Basalmembranproteine erhält sowie Proteasehinhibitoren und Chelatbildner enthält,
die Vorextraktion oder Extraktion nicht jedoch in Anwesenheit von Triton® X 100 erfolgt.

2. Verwendung nach Anspruch 1, dadurch gekennzeichnet, daß unter Zugabe von chelatbildenden organischen Polysäuren extrahiert wird.

3. Verwendung nach Anspruch 2, dadurch gekennzeichnet, daß unter Zugabe von EDTA oder EGTA extrahiert wird.

4. Verwendung nach einem oder mehreren der Ansprüche 1 bis 3, dadurch gekennzeichnet, daß unter Zugabe von 1 bis 500 mM des Chelatbildners extrahiert wird.

5. Verwendung nach Anspruch 4, dadurch gekennzeichnet, daß unter Zugabe von 10 bis 50 mM des Chelatbildners extrahiert wird.

6. Verwendung der nach einem oder mehreren der Ansprüche 1 bis 5 erhältlichen Basalmembranproteine zur Herstellung hochspezifischer Antiseren oder Antikörper und damit zur Bestimmung der Basalmembranproteine in Körperflüssigkeiten und Gewebeextrakten.

## Claims

1. The use of chelating agents for the isolation of basement membrane proteins from human or animal tissues, which comprises the following steps:
a) a preextraction of the tissue which contains basement membrane with a buffer which maintains the biological activity of the basement membrane proteins and contains protease inhibitors;
b) an extraction of the basement membrane proteins from human or animal tissues using a buffer which maintains the biological activity of the basement membrane proteins and contains protease inhibitors and chelating agents,
but the preextraction or extraction does not take place in the presence of Triton® X 100.

2. The use as claimed in claim 1, wherein extraction is carried out with the addition of chelate-forming organic polyacids.

3. The use as claimed in claim 2, wherein extraction is carried out with the addition of EDTA or EGTA.

4. The use as claimed in one or more of claims 1 to 3, wherein extraction is carried out with the addition of 1 to 500 mM of the chelating agent.

5. The use as claimed in claim 4, wherein extraction is carried out with the addition of 10 to 50 mM of the chelating agent.

6. The use of the basement membrane proteins obtainable as claimed in one or more of claims 1 to 5, for the preparation of highly specific antisera or antibodies and hence for the determination of basement membrane proteins in body fluids and tissue extracts.

## Revendications

1. Utilisation d'agents chélateurs pour l'isolement de protéines de membrane basale à partir de tissus humains ou animaux, comportant les étapes suivantes:
a) une pré-extraction du tissu contenant les membranes basales, à l'aide d'un tampon qui maintient l'activité biologique des protéines de membrane basale et contient des inhibiteurs de protéases;
b) une extraction des protéines de membrane basale à partir de tissus humains ou animaux, à l'aide d'un tampon qui maintient l'activité biologique des protéines de membrane basale et contient des inhibiteurs de protéases et des agents chélateurs,
la pré-extraction ou l'extraction ne s'effectuant toutefois pas en présence de Triton® X 100.

2. Utilisation selon la revendication 1, caractérisée en ce que l'on effectue l'extraction avec addition de polyacides organiques chélateurs.

3. Utilisation selon la revendication 2, caractérisée en ce que l'on effectue l'extraction avec addition d'EDTA ou d'EGTA.

4. Utilisation selon une ou plusieurs des revendications 1 à 3, caractérisée en ce que l'on effectue l'extraction avec addition de 1 à 500 mM de l'agent chélateur.

5. Utilisation selon la revendication 4, caractérisée en ce que l'on effectue l'extraction avec addition de 10 à 50 mM de l'agent chélateur.

6. Utilisation des protéines de membrane basale, pouvant être obtenues selon une ou plusieurs des revendications 1 à 5, pour la production d'anticorps ou d'antisérums hautement spécifiques et ainsi pour la détermination des protéines de membrane basale dans des liquides corporels et des extraits tissulaires.
